# EUROPÄISCHE PATENTSCHRIFT

(11) **EP 2 110 086 B1**
(45) Veröffentlichungstag und Bekanntmachung des Hinweises auf die Patenterteilung: **03.08.2011**
(21) Anmeldenummer: 09157162.0
(22) Anmeldetag: 02.04.2009
(51) Int. Cl.: A61B 17/225

(54) **Medizinischer Arbeitsplatz**
Medical workstation
Poste de travail médical

(30) Priorität: 18.04.2008 DE 102008019635
(43) Veröffentlichungstag der Anmeldung: 21.10.2009
(73) Patentinhaber: Siemens Aktiengesellschaft, 80333 München (DE)
(72) Erfinder: Mahler, Matthias, 91058 Erlangen (DE)

(56) Entgegenhaltungen:
- EP-A- 1 506 741
- DE-A1-102004 010 466
- DE-C1- 4 003 350

## Beschreibung

Die Erfindung betrifft einen medizinischen Arbeitsplatz mit einer Diagnoseeinheit, einer Therapieeinheit und einem Behandlungstisch, welche mit Hilfe einer Positioniervorrichtung relativ zueinander positionierbar sind. Ein solcher medizinischer Arbeitsplatz ist beispielsweise aus der DE 10 2004 010 466 A1 bekannt.

Moderne medizinische Arbeitsplätze werden für eine Vielzahl von verschiedenen Untersuchungen oder Behandlungen genutzt. Aus diesem Grund ist es wünschenswert, Diagnose- und Therapieeinheiten flexibel miteinander kombinieren zu können.

Auch medizinische Arbeitsplätze zur extrakorporalen Stoßwellenlithotripsie werden zunehmend flexibel gestaltet. Die verwendeten Einheiten, üblicherweise ein Lithotripter, ein Röntgen C-Bogen-Gerät und ein Behandlungstisch, werden zur Durchführung einer Steinbehandlung miteinander kombiniert, insbesondere dann, wenn lediglich eine geringe Anzahl von Steinbehandlungen durchgeführt wird. In der Zwischenzeit können die Geräte anderweitig verwendet werden.

Eine erfolgreiche und sichere Lithotripsie, beispielsweise von Nierensteinen, setzt voraus, dass der Lithotripter, das Röntgen C-Bogen-Gerät und der Patiententisch exakt zueinander positioniert sind.

Die Justage der in dem medizinischen Arbeitsplatz vorhandenen Geräte gliedert sich in eine Vorpositionierung (von Röntgen C-Bogen-Gerät, Lithotripter und Behandlungstisch) und eine anschließende Feinjustage (des Röntgen C-Bogens auf den Stoßwellenfokus des Lithotripters). Im Rahmen der Vorpositionierung werden die gesamten Einheiten relativ zueinander verschoben. Beispielsweise wird der Patiententisch gegenüber dem Lithotripter verschoben. Bei der Feinjustage werden lediglich Teile der Geräte, beispielsweise der C-Bogen des Röntgengerätes verfahren. Eine genaue Vorpositionierung sichert maximale Verfahrwege bei der Therapie.

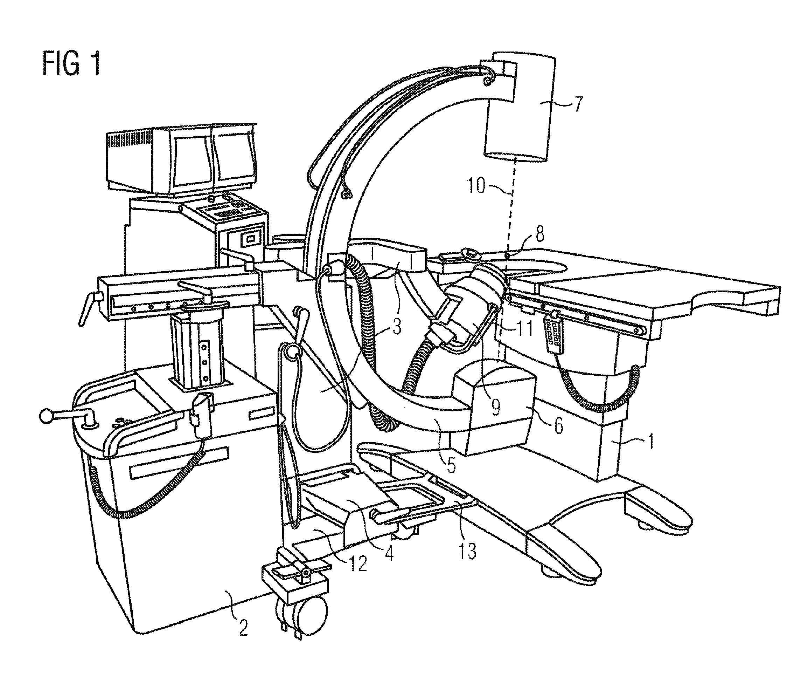

Figur 1 zeigt einen allgemein bekannten medizinischen Arbeitsplatz mit einem Behandlungstisch 1, einem Röntgen C-Bogen-Gerät 2 und einem Lithotripter 3. Die Einheiten sind mit Hilfe einer zentralen mechanischen Schnittstelle 4 miteinander verbunden. Diese Schnittstelle wird üblicherweise von einem Servicetechniker justiert und auf den Ausleger des Röntgen C-Bogen-Geräts geklemmt. Das Röntgen C-Bogen-Gerät 2 umfasst einen C-Bogen 5, dessen unteres Ende eine Röntgenstrahlquelle 6 und dessen oberes Ende einen Detektor 7 trägt. Vor der Durchführung einer Steinbehandlung ist der Zentralstrahl 10 der Röntgenstrahlquelle 6 so einzujustieren, dass er den Fokuspunkt 8 des Stoßwellenkopfes 9 trifft. Der Fokuspunkt 8 und das Isozentrum des Röntgen C-Bogen-Gerätes 2 fallen idealerweise zusammen, oder liegen zumindest nahe beieinander. Zur Erleichterung der Justage zwischen dem Röntgen C-Bogen-Gerät 2 und dem Lithotripter 3 kann eine Justagehilfe verwendet werden. Zu diesem Zweck befindet sich beispielsweise an dem Stoßwellenkopf 9 ein klappbarer Bügel 11, welcher den Fokuspunkt 8 des Stoßwellenkopfes 9, mit einer in der Röntgendarstellung sichtbaren Kugel, markiert.

Zur Vorpositionierung des Behandlungstisches 1 gegenüber dem Röntgen C-Bogen-Gerät 2 weist dieser eine Vorrichtung in Form einer Gabel 13 auf, die die auf einem Ausleger 12 des Röntgen C-Bogen-Gerät 2 montierte mechanische Schnittstelle 4 formschlüssig kontaktiert. Gleiches trifft für den Lithotripter 3 zu, dessen Gabel nicht näher dargestellt ist. Jedes der drei auszurichtenden Geräte weist somit eine Vorrichtung (z.B. eine Schnittstelle 4 oder eine Gabel 13) auf, die der mechanischen Kopplung und Vorpositionierung der Geräte untereinander dient.

Offensichtlich kommt der mechanischen Schnittstelle eine zentrale Rolle bei der Vorpositionierung zu. Eine exakte Positionierung der Schnittstelle auf dem Ausleger des Röntgen C-Bogen-Geräts ist daher wichtig. Auf diese Weise wird sichergestellt, dass während der Therapie der zu behandelnde Stein stets im Röntgenbild des Röntgen C-Bogen-Gerätes 2 dargestellt werden kann. Um diese Justage nicht zu verlieren, darf die mechanische Schnittstelle nicht von dem Ausleger entfernt werden. Die auf dem Ausleger somit dauerhaft vorhandene mechanische Schnittstelle, erweist sich jedoch für anderweitige Verwendungen des Röntgen C-Bogen-Gerätes vielfach als hinderlich.

Aufgabe der vorliegenden Erfindung ist es, einen medizinischen Arbeitsplatz mit einer Diagnoseeinheit, einer Therapieeinheit und einem Behandlungstisch anzugeben, die mit Hilfe einer Positioniervorrichtung auf einfache Weise zueinander positionierbar sind. Außerdem soll ein Verfahren zur vereinfachten Positionierung einer Diagnoseeinheit, einer Therapieeinheit und eines Behandlungstisches in einem medizinischen Arbeitsplatz angegeben werden.

Hinsichtlich der Vorrichtung wird die Aufgabe gemäß der Erfindung gelöst durch einen medizinischen Arbeitsplatz mit den Merkmalen nach Anspruch 1.

Der erfindungsgemäße medizinische Arbeitsplatz umfasst eine Diagnoseeinheit, eine Therapieeinheit, einen Behandlungstisch und eine Positioniervorrichtung. Mit Hilfe der Positioniervorrichtung sind die Diagnoseeinheit, die Therapieeinheit und der Behandlungstisch in dem medizinischen Arbeitsplatz relativ zueinander positionierbar. Die Positioniervorrichtung umfasst einen ersten und einen zweiten Ausleger, die zwischen einer Ruhe- und einer vorgebbaren Arbeitsposition bewegbar sind. Dabei dient der erste Ausleger in seiner Arbeitsposition der relativen Positionierung zwischen Behandlungstisch und Therapieeinheit. Der erste Ausleger ist an dem Behandlungstisch oder der Therapieeinheit angeordnet, und weist ein erstes Strukturelement auf, welches zur Vorgabe einer Sollposition der Therapieeinheit bzw. des Behandlungstisches dient. Der zweite Ausleger der Positioniervorrichtung dient in seiner Arbeitsposition der relativen Positionierung zwischen der Diagnoseeinheit einerseits und Behandlungstisch oder Therapieeinheit andererseits. Der zweite Ausleger ist an dem Behandlungstisch oder der Therapieeinheit angeordnet, und weist ein zweites Strukturelement auf, welches zur visuellen Anzeige einer Sollposition der Diagnoseeinheit dient. Die Diagnoseeinheit umfasst ihrerseits eine sichtbare Markierung zur visuellen Positionierung bezüglich des vorgenannten zweiten Strukturelementes.

Mit einer solchen Positioniervorrichtung ist eine einfache und schnelle Vorpositionierung zwischen Diagnoseeinheit, Therapieeinheit und Behandlungstisch möglich. Somit sind die optimalen Verfahrwege für die Durchführung von diagnostischen oder therapeutischen Maßnahmen garantiert. Da die Positioniervorrichtung lediglich zwei Ausleger umfasst, kann gegenüber bekannten mechanischen Schnittstellen ein Bauteil eingespart werden. Da außerdem die beiden Ausleger der Positioniervorrichtung zwischen einer Ruhe- und einer vorgebbaren Arbeitsposition bewegbar sind, weisen die Einheiten keine störenden Anbauten auf, die ihren Einsatz in anderen medizinischen Arbeitsplätzen gegebenenfalls behindern. Dies ist insbesondere für die Diagnoseeinheit vorteilhaft, da die zum Verbleib auf ihrem Ausleger bestimmte mechanische Schnittstelle entfällt, und durch eine Markierung ersetzt wird. Die Diagnoseeinheit ist mit Hilfe einer visuellen Anzeige und einer sichtbaren Markierung bezüglich der Therapieeinheit bzw. dem Patiententisch positionierbar. Da keine Kräfte übertragen werden müssen, ist es möglich, den zweiten Ausleger auf geringe mechanische Belastungen hin auszulegen. Dies führt zu einem Gewinn an Stauraum sowie zu Gewichtseinsparungen an der betreffenden Einheit. Durch den Wegfall einer zentralen mechanischen Schnittstelle können die Ausleger der Positioniervorrichtung nahezu an beliebiger Stelle an den Einheiten angebracht werden. Die Ausleger können an solchen Stellen angebracht werden, an denen sie als wenig störend empfunden werden. Insbesondere kann vermieden werden, dass die Ausleger im Bereich einer Aussparung des Patiententisches, die dem Durchgriff eines Stoßwellenkopfes der Therapieeinheit dient, angebracht werden. Dies ist insbesondere vorteilhaft, da im Bereich der Aussparung des Patiententisches, unterhalb dessen Liegefläche, üblicherweise die Röntgenstrahlquelle des Röntgen C-Bogen-Gerätes angeordnet ist. Bei konventionellen Arbeitsplätzen besteht die Gefahr, dass die mechanische Schnittstelle und die Röntgenstrahlquelle in diesem Bereich miteinander kollidieren. Das Risiko einer solchen Kollision kann wird hierdurch deutlich verringert.

Nach einer ersten Ausführungsform ist der erste Ausleger an dem Behandlungstisch angeordnet. Das Strukturelement des ersten Auslegers ist durch eine Anschlagfläche gebildet. Zur Positionierung der Therapieeinheit gegenüber dem Behandlungstisch tritt beispielsweise ein bestimmter Teil des Gehäuses der Therapieeinheit mit der Anschlagfläche des ersten Auslegers in mechanischen Kontakt. Dabei kann die Therapieeinheit gemäß zweier Weiterbildungen sowohl unmittelbar als auch mittelbar in mechanischen Kontakt mit der Anschlagfläche treten. Der mittelbare Kontakt tritt durch Kontakt der Anschlagfläche des ersten Auslegers mit einer weiteren Anschlagfläche des an der Therapieeinheit angeordneten zweiten Auslegers ein. Sowohl bei der Therapieeinheit als auch bei dem Behandlungstisch handelt es sich um relativ schwere Einheiten, deren Positionierung mit Hilfe eines mechanischen Anschlages deutlich erleichtert werden kann. Ein direkter Anschlag, beispielsweise an einer Kante des Gehäuses, ist besonders einfach und wenig fehleranfällig. Ein mittelbarer Anschlag erlaubt eine weitgehend freie Ausgestaltung der Anschlagflächen. Beispielsweise können die die Anschlagflächen ausbildenden Teilstücke des ersten und zweiten Auslegers ihrer Form nach aufeinander abgestimmt sein. So können diese insbesondere nach einen Stecker-Steckdose-Prinzip ineinandergreifen, so dass Fehlbedienungen ausgeschlossen sind.

Nach einer alternativen Ausführungsform ist der erste Ausleger nicht an dem Behandlungstisch, sondern an der Therapieeinheit angeordnet. Das Strukturelement des ersten Auslegers ist nun so ausgestaltet, dass die Sollposition des Behandlungstisches visuell vorgegeben ist. Der Behandlungstisch weist eine zweite sichtbare Markierung auf, mit deren Hilfe er bezüglich der Therapieeinheit, genauer bezüglich des Strukturelementes des ersten Auslegers, positionierbar ist. Bei einem solchen Strukturelement zur visuellen Vorgabe einer Sollposition kann es sich beispielsweise um eine Peilvorrichtung, ein Fadenkreuz oder einen Lichtzeiger, wie beispielsweise einen Laserpointer handeln. Der Behandlungstisch kann ebenfalls anhand einer strich- oder kreuzförmigen Markierung, die entlang einer Kante des ersten Auslegers ausgerichtet wird, positioniert werden. Da der Behandlungstisch lediglich eine sichtbare Markierung, d.h. keinen Ausleger aufweist, kann eine Vielzahl von Behandlungstischen in einen solchen medizinischen Arbeitsplatz, durch Anbringen entsprechender Markierungen, integriert werden, ohne dass diese aufwändigen Veränderungen unterzogen werden müssen.

Nach einer weiteren Ausführungsform sind beide Ausleger der Positioniervorrichtung an dem Behandlungstisch angeordnet. In diesem Fall kann vermieden werden, dass die Therapieeinheit Ausleger aufweist. Somit steht an dieser Einheit mehr Stauraum zur Verfügung, beispielsweise für Kabel. Nach einer alternativen Ausführungsform sind die beiden Ausleger an der Therapieeinheit angeordnet. Somit können jegliche Ausleger an dem Behandlungstisch vermieden werden, so dass dieser flexibel auch in anderen medizinischen Arbeitsplätzen eingesetzt werden kann.

Bei der Therapieeinheit handelt es sich vorzugsweise um einen Lithotripter. Moderne Anlagen zur Lithotripsie sind mobil und daher flexibel einsetzbar. Die Möglichkeit einen medizinischen Arbeitsplatz flexibel zusammenzustellen ist daher besonders bei der Lithotripsie vorteilhaft. Bei dem Diagnosegerät handelt es sich bevorzugt um ein Röntgen C-Bogen-Gerät. Solche Röntgengeräte werden für eine Vielzahl von verschiedenen Untersuchungen verwendet, entsprechend viele verschiedene Modelle sind am Mark erhältlich oder bereits im Einsatz. Nach der vorstehenden Ausführungsform wird dem Wunsch entsprochen, solche Röntgengeräte variabel in ein Lithotripsiesystem zu integrieren.

Verfahrensbezogen wird die Aufgabe durch ein Verfahren mit den Merkmalen nach Anspruch 10 gelöst.

Das erfindungsgemäße Verfahren zur Positionierung einer Therapieeinheit, einer Diagnoseeinheit und eines Behandlungstisches in einem medizinischen Arbeitsplatz, relativ zueinander, weist die folgenden Schritte auf: Behandlungstisch und Therapieeinheit werden zueinander positioniert, indem ein an dem Behandlungstisch oder der Therapieeinheit angeordneter erster Ausleger von seiner Ruhe- in seine vorgegebene Arbeitsposition verbracht wird. Anschließend wird mit Hilfe eines an dem ersten Ausleger angeordneten Strukturelementes die Sollposition der Therapieeinheit bzw. des Behandlungstisches vorgegeben und die Therapieeinheit bzw. der Behandlungstisch an die vorgegebene Sollposition verbracht. Die Diagnoseeinheit wird relativ zu dem Behandlungstisch oder der Therapieeinheit positioniert. Dies geschieht, indem zunächst ein an dem Behandlungstisch oder der Therapieeinheit angeordneter zweiter Ausleger von seiner Ruhe- in seine Arbeitsposition verbracht wird. Mit Hilfe eines an dem zweiten Ausleger angeordneten zweiten Strukturelementes wird die Sollposition der Diagnoseeinheit visuell angezeigt. Anhand einer auf der Diagnoseeinheit vorhandenen sichtbaren Markierung wird diese bezüglich des zweiten Strukturelementes positioniert.

Wesentliche Vorteile des erfindungsgemäßen Verfahrens sind bereits im Zusammenhang mit dem erfindungsgemäßen medizinischen Arbeitsplatzes genannt. Vorteilhafte Weiterbildungen des erfindungsgemäßen Verfahrens gehen aus den von Anspruch 10 abhängigen Ansprüchen hervor.

Nach einer Ausführungsform des erfindungsgemäßen Verfahrens erfolgt die Positionierung zwischen Behandlungstisch und Therapieeinheit zeitlich gesehen vor der Positionierung der Diagnoseeinheit relativ zu dem Behandlungstisch oder der Therapieeinheit. Werden zunächst Therapieeinheit und Behandlungstisch relativ zueinander positioniert, so ist sichergestellt, dass der Diagnoseeinheit maximale Verfahrwege zur Verfügung stehen.

Nach einer weiteren Ausführungsform werden nach erfolgter Positionierung Therapieeinheit, Diagnoseeinheit und Behandlungstisch in dem medizinischen Arbeitsplatz fixiert. Üblicherweise sind die Einheiten auf Rollen gelagert. Die Fixierung geschieht beispielsweise durch Blockieren der Rollen mit allgemein bekannten Mitteln, wie etwa Rasten oder Klemmvorrichtungen. Es konnte herausgefunden werden, dass eine kraftschlüssige mechanische Verbindung der einzelnen Geräte untereinander nicht notwendig ist, so dass auf diese verzichtet werden kann. Es ist ausreichend, wenn die vorgenannten Einheiten nach erfolgter Positionierung fixiert werden. Mit anderen Worten werden die Einheiten gegen unbeabsichtigtes Verfahren gesichert, d.h. gebremst.

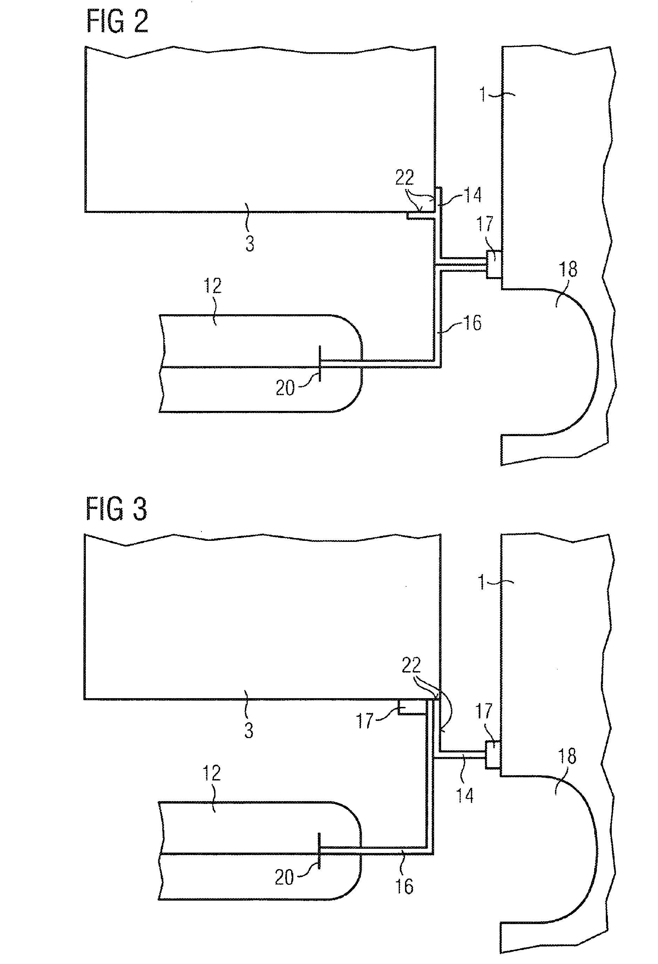

Weitere vorteilhafte Ausgestaltungen des erfindungsgemäßen medizinischen Arbeitsplatzes sowie des erfindungsgemäßen Verfahrens gehen aus den vorstehend nicht angesprochenen Unteransprüchen hervor. Zur weiteren Erläuterung der Erfindung wird auf die Ausführungsbeispiele in den Figuren 2 bis 5 der Zeichnung verwiesen.

Es zeigen:
Figur 1 einen medizinischen Arbeitsplatz gemäß dem Stand der Technik,
Figuren 2 bis 5 schematische Detailansichten erfindungsgemäβer Ausgestaltungen eines solchen medizinischen Arbeitsplatzes.

Ein aus dem Stand der Technik bekannter medizinischer Arbeitsplatz mit einem Behandlungstisch 1, einem Röntgen C-Bogen-Gerät 2 als Diagnoseeinheit und einem Lithotripter 3 als Therapieeinheit ist aus Figur 1 bekannt.

Figur 2 zeigt ein erstes Ausführungsbeispiel eines verbesserten medizinischen Arbeitsplatzes in einer Detailansicht. Gezeigt ist ein Behandlungstisch 1, ein Lithotripter 3 und ein Röntgen C-Bogen-Gerät 2, in einer Draufsicht. Die in dem Behandlungstisch 1 vorhandene Aussparung 18 dient dem Durchgriff des Stoßwellenkopfes 9 des Lithotripters 3. An dem Behandlungstisch 1 befindet sich ein erster und zweiter Ausleger 14, 16, die jeweils mit Hilfe eines Scharniers 17 an dem Behandlungstisch 1 befestigt sind. Die Ausleger 14, 16 sind Teil einer Positioniervorrichtung, die die allgemein aus dem Stand der Technik bekannte, in F-gur 1 dargestellte, mechanische Schnittstelle 4 einschließlich der dazugehörigen Gabel 13 ersetzt. Die Ausleger 14, 16 sind zwischen einer Ruhe-und einer Arbeitsposition bewegbar. Figur 2 zeigt die beiden Ausleger 14, 16 in ihrer Arbeitsposition. Der erste Ausleger 14 gibt die Position zwischen Behandlungstisch 1 und Lithotripter 3 vor. Zur Positionierung wird der Lithotripter 3, genauer ein Teil seines Gehäuses, mit der endseitig an dem ersten Ausleger 14 vorhandenen Anschlagflächen 22 in Kontakt gebracht. Da der erste Ausleger 14 bei dem Positioniervorgang nicht zu vernachlässigende mechanische Kräfte aufnimmt, ist er beispielsweise aus Stahl gefertigt. Nach erfolgter Positionierung werden Behandlungstisch 1 und Lithotripter 3 in ihrer Position fixiert, d.h. gegen unbeabsichtigtes Verfahren gesichert.

Der zweite Ausleger 16, welcher in gleicher Weise wie der erste Ausleger 14 zwischen einer Ruhe- und einer Arbeitsposition schwenkbar ist, und in Figur 2 in seiner Arbeitsposition dargestellt ist, dient zur Positionierung des Röntgen C-Bogen-Gerätes 2 gegenüber dem Behandlungstisch 1. Zu diesem Zweck weist der Ausleger 12 eine Markierung 20 in Form eines langgestreckten Kreuzes auf, die bezüglich des Freiendes des zweiten Auslegers 16 ausgerichtet wird. Da der zweite Ausleger 16 lediglich der visuellen Anzeige der gewünschten Position des Röntgen C-Bogen-Gerätes 2 dient, kann dieser verglichen mit dem ersten Ausleger 14 mechanisch weniger belastbar ausgelegt sein. Dieser kann beispielsweise aus Leichtmetall oder aus Kunststoff gefertigt sein.

In einem medizinischen Arbeitsplatz können Einheiten (Behandlungstisch 1, Röntgen C-Bogen-Gerät 2 und Lithotripter 3) verschiedener Hersteller, die sehr unterschiedlich aufgebaut sind, beispielsweise unterschiedliche Gehäuse aufweisen, miteinder kombiniert werden. Beispielsweise können mit ein und demselben Behandlungstisch 1 wahlweise verschiedene Röntgen C-Bogen-Geräte 2 unterschiedlicher Hersteller kombiniert werden. Die durch die beiden Ausleger 14, 16 zwischen den einzelnen Einheiten vorgebbaren Abstände, variieren abhängig von der jeweiligen Konfiguration, in der die Einheiten miteinander kombiniert werden. Aus diesem Grund kann der erste und/oder zweite Ausleger 14, 16 in der Länge verstellbar sein. Beispielsweise kann die Länge der Schenkel der Ausleger 14, 16 teleskopartig veränderbar sein.

In einem weiteren Ausführungsbeispiel gemäß Figur 3 befindet sich der zweite Ausleger 16 an dem Lithotripter 3 angebracht. Der zweite Ausleger 16 ist dort ebenfalls mit Hilfe eines Scharniers 17 klappbar montiert. Das Freiende des zweiten Auslegers 16 zeigt, in gleicher Weise wie im Zusammenhang mit Figur 2 erläutert, auf visuellem Wege die Sollposition des Auslegers 12 des Röntgen C-Bogen-Gerätes 2 im Zusammenspiel mit einer Markierung 20 an. Bei einer solchen Markierung 20 kann es sich beispielsweise um ein Etikett oder eine farbliche Markierung handeln. Zur Positionierung zwischen Behandlungstisch 1 und Lithotripter 3 schlägt der erste Ausleger 14 mit seinem stirnseitigen Freiende an das Gehäuse des Lithotripters 3 und mit einer Flanke an den zweiten Ausleger 16 an. Durch diese beiden Anschlagflächen 22 ist die Position des Lithotripters 3 bezüglich des Behandlungstisches 1 vorgegeben.

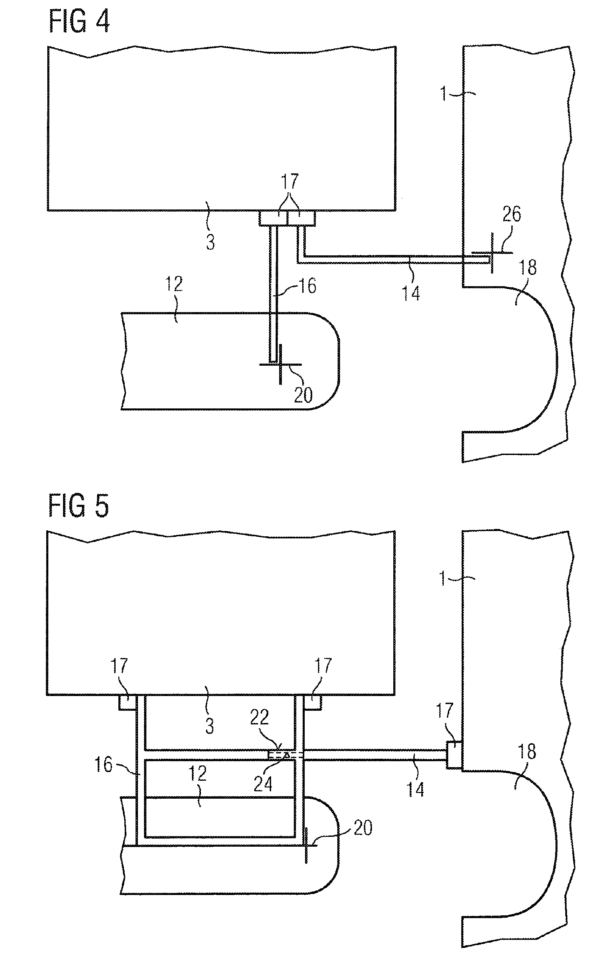

Nach dem in Figur 4 gezeigten Ausführungsbeispiel sind beide Ausleger 14, 16 an dem Lithotripter 3 angebracht. Ihre Freienden zeigen jeweils visuell die Sollpositionen von Röntgen C-Bogen-Gerät 2 und Behandlungstisch 1 an. Dabei wirken die Freienden mit den entsprechenden Markierungen 20 und 26 zusammen, die an dem Ausleger 12 bzw. dem Behandlungstisch 1 angebracht sind. Die auf visuellem Wege erfolgende Positionierung kann neben der in Figur 4 dargestellten Möglichkeit auch dadurch erfolgen, dass die Ausleger 14, 16 ein Fadenkreuz, eine Peilvorrichtung oder einen Lichtzeiger, etwa einen Laserpointer, als Strukturelement aufweisen. Das entsprechende Strukturelement, beispielsweise ein Lichtzeiger, bzw. dessen projizierter Lichtpunkt wird mit einer entsprechenden Gegenmarkierung in Übereinstimmung gebracht wird. Die Ausleger 14, 16 sind in ihrer Arbeitsposition dargestellt und mit Hilfe entsprechend geeigneter Scharniere 17 in ihre Ruheposition zurückschwenkbar.

Die Form des ersten und zweiten Auslegers 14, 16 kann relativ frei gewählt werden. Entscheidend ist, dass die Ausleger 14, 16 die gewünschten Sollpositionen anzeigen, bzw. eine Anschlagfläche 22, 24 zur Verfügung stellen, durch die die gewünschte Sollposition vorgegeben ist. So zeigt Figur 5 ein weiteres Ausführungsbeispiel, bei dem der zweite Ausleger 16 aus Längs- und Querverstrebungen aufgebaut ist. Bei den Verstrebungen, die den zweiten Ausleger 16 bilden, kann es sich insbesondere um Metallhohlprofile handeln. Der zweite Ausleger 16 weist auf seiner dem ersten Ausleger 14 zugewandten Seite eine Zugangsöffnung auf, so dass der erste Ausleger 14 mit seinem Freiende in einen entsprechenden Teil des zweiten Auslegers 16 eingreifen kann. Dabei schlagen der erste und zweite Ausleger 14, 16 mit ihren Anschlagflächen 22 und 24 formschlüssig aneinander an. Die Ausleger 14, 16 können auβerdem mit Vorrichtungen versehen sein, die eine mechanische Verriegelung zwischen den beiden Auslegern 14, 16 ermöglichen. Dies kann beispielsweise mit Hilfe von Rastelementen erfolgen. Das Röntgen C-Bogen-Gerät 2 wird anhand entsprechender Markierungen 20, die sich auf dem Ausleger 12 befinden, bezüglich Behandlungstisch 1 und Lithotripter 3 ausgerichtet.

## Patentansprüche

1. Medizinischer Arbeitsplatz mit einer Diagnoseeinheit (2), einer Therapieeinheit (3), einem Behandlungstisch (1) und einer Positioniervorrichtung mit deren Hilfe der Behandlungstisch (1), die Diagnose- und die Therapieeinheit (2, 3) relativ zueinander positionierbar sind, mit den folgenden Merkmalen:
a) die Positioniervorrichtung umfasst einen ersten und einen zweiten Ausleger (14, 16), die zwischen einer Ruhe- und einer vorgebbaren Arbeitsposition bewegbar sind,
**dadurch gekennzeichnet, dass**
b) der erste Ausleger (14) dient in seiner Arbeitsposition der relativen Positionierung zwischen Behandlungstisch (1) und Therapieeinheit (3), ist an dem Behandlungstisch (1) oder der Therapieeinheit (3) angeordnet, und weist ein erstes Strukturelement zur Vorgabe einer Sollposition der Therapieeinheit (3) bzw. des Behandlungstisches (1) auf,
c) der zweite Ausleger (16) dient in seiner Arbeitsposition der relativen Positionierung zwischen der Diagnoseeinheit (2) einerseits und dem Behandlungstisch (1) oder der Therapieeinheit (3) andererseits, ist an dem Behandlungstisch (1) oder der Therapieeinheit (3) angeordnet, und weist ein zweites Strukturelement zur visuellen Anzeige einer Sollposition der Diagnoseeinheit (2) auf,
d) die Diagnoseeinheit (2) umfasst eine sichtbare Markierung für ihre visuelle Positionierung bezüglich des zweiten Strukturelementes.

2. Medizinischer Arbeitsplatz nach Anspruch 1, bei dem
- der erste Ausleger (14) an dem Behandlungstisch (1) angeordnet ist
- das Strukturelement des ersten Auslegers (14) durch eine Anschlagfläche (22) gebildet ist, die zur Positionierung der Therapieeinheit (3) gegenüber dem Behandlungstisch (1) durch einen mechanischen Kontakt dient.

3. Medizinischer Arbeitsplatz nach Anspruch 2, bei dem die Therapieeinheit (3) und die Anschlagfläche (22) des ersten Auslegers (14) dazu vorgesehen sind unmittelbar mechanisch aneinander anzuschlagen.

4. Medizinischer Arbeitsplatz nach einem der vorstehenden Ansprüche, bei dem der erste und der zweite Ausleger (14, 16) an dem Behandlungstisch (1) angeordnet sind.

5. Medizinischer Arbeitsplatz nach Anspruch 2, bei dem die Therapieeinheit (3) und der erste Ausleger (14) dazu vorgesehen sind mittelbar - durch Anschlag der Anschlagfläche (22) des ersten Auslegers (14) an einer weiteren Anschlagfläche (24) des an der Therapieeinheit (3) angeordneten zweiten Auslegers (16) - aneinander anzuschlagen.

6. Medizinischer Arbeitsplatz nach Anspruch 1, bei dem
- der erste Ausleger (14) an der Therapieeinheit (3) angeordnet ist,
- das Strukturelement des ersten Auslegers (14) zur visuellen Vorgabe einer Sollposition des Behandlungstisches (1) ausgestaltet ist und
- der Behandlungstisch (1) eine weitere sichtbare Markierung (26) zur visuellen Positionierung des Behandlungstisches (1) bezüglich der Therapieeinheit (3) umfasst.

7. Medizinischer Arbeitsplatz nach Anspruch 1 oder 6, bei dem der erste und der zweite Ausleger (14, 16) an der Therapieeinheit (3) angeordnet sind.

8. Medizinischer Arbeitsplatz nach einem der vorstehenden Ansprüche, bei dem die Therapieeinheit (3) ein Lithotripter ist.

9. Medizinischer Arbeitsplatz nach einem der vorstehenden Ansprüche, bei dem das Diagnosegerät (2) ein Röntgen C-Bogen Gerät ist.

10. Verfahren zur Positionierung einer Therapieeinheit (3), einer Diagnoseeinheit (2) und eines Behandlungstisches (1) in einem medizinischen Arbeitsplatz relativ zueinander, mit den folgenden Schritten:
a) Positionieren des Behandlungstisches (1) und der Therapieeinheit (3) relativ zueinander, indem
- ein an dem Behandlungstisch (1) oder der Therapieeinheit (3) angeordneter erster Ausleger (14) von seiner Ruhein seine vorgegebene Arbeitsposition verbracht wird,
- mit Hilfe eines an dem ersten Ausleger (14) angeordneten Strukturelementes die Sollposition der Therapieeinheit (3) bzw. des Behandlungstisches (1) vorgegeben wird und
- die Therapieeinheit (3) bzw. der Behandlungstisches (1) an die vorgegebene Sollposition verbracht wird,
b) Positionieren der Diagnoseeinheit (2) relativ zu dem Behandlungstisch (1) oder der Therapieeinheit (3), indem
- ein an dem Behandlungstisch (1) oder der Therapieeinheit (3) angeordneter zweiter Ausleger (16) von seiner Ruhein seine Arbeitsposition verbracht wird,
- mit Hilfe eines an dem zweiten Ausleger (16) angeordneten zweiten Strukturelementes die Sollposition der Diagnoseeinheit (2) visuell angezeigt wird und
- die Diagnoseeinheit (2) anhand einer an dieser vorhandenen sichtbaren Markierung bezüglich des zweiten Strukturelementes positioniert wird.

11. Verfahren nach Anspruch 10, bei dem die Positionierung zwischen Behandlungstisch (1) und Therapieeinheit (3) zeitlich gesehen vor der Positionierung zwischen der Diagnoseeinheit (2) relativ zu dem Behandlungstisch (1) oder der Therapieeinheit (3) erfolgt.

12. Verfahren nach Anspruch 10 oder 11, bei dem nach erfolgter Positionierung von Behandlungstisch (1) und Therapieeinheit (3) diese in dem medizinischen Arbeitsplatz fixiert werden.

## Claims

1. Medical workstation with a diagnosis unit (2), a therapy unit (3), a treatment table (1) and a positioning device, by means of which the treatment table (1), the diagnosis unit and the therapy unit (2, 3) can be positioned in relation to one another, comprising the following features:
a) the positioning device comprises a first and a second arm (14, 16), which can be moved between a rest position and a predeterminable operational position,
**characterized in that**
b) the first arm (14), in its operational position, serves for the relative positioning between treatment table (1) and therapy unit (3), is arranged on the treatment table (1) or on the therapy unit (3) and comprises a first structural element for predetermining an intended position of the therapy unit (3) or the treatment table (1),
c) the second arm (16), in its operational position, serves for the relative positioning between, firstly, the diagnosis unit (2) and, secondly, the treatment table (1) or the therapy unit (3), is arranged on the treatment table (1) or the therapy unit (3) and comprises a second structural element for visually displaying an intended position of the diagnosis unit (2),
d) the diagnosis unit (2) comprises a visible marking for its visual positioning in relation to the second structural element.

2. Medical workstation according to Claim 1, wherein
- the first arm (14) is arranged on the treatment table (1)
- the structural element of the first arm (14) is formed by an abutment surface (22) that serves for positioning the therapy unit (3) in relation to the treatment table (1) by mechanical contact.

3. Medical workstation according to Claim 2, wherein the therapy unit (3) and the abutment surface (22) of the first arm (14) are provided to abut directly against one another in a mechanical fashion.

4. Medical workstation according to one of the preceding claims, wherein the first and the second arm (14, 16) are arranged on the treatment table (1).

5. Medical workstation according to Claim 2, wherein the therapy unit (3) and the first arm (14) are provided to abut indirectly against one another, by the abutment surface (22) of the first arm (14) abutting against a further abutment surface (24) of the second arm (16) arranged on the therapy unit (3).

6. Medical workstation according to Claim 1, wherein
- the first arm (14) is arranged on the therapy unit (3),
- the structural element of the first arm (14) is embodied for visually predetermining an intended position of the treatment table (1) and
- the treatment table (1) comprises a further visible marking (26) for the visual positioning of the treatment table (1) in relation to the therapy unit (3).

7. Medical workstation according to Claim 1 or 6, wherein the first and the second arm (14, 16) are arranged on the therapy unit (3).

8. Medical workstation according to one of the preceding claims, wherein the therapy unit (3) is a lithotriptor.

9. Medical workstation according to one of the preceding claims, wherein the diagnosis unit (2) is an X-ray C-arc device.

10. Method for positioning a therapy unit (3), a diagnosis unit (2) and a treatment table (1) relative to one another in a medical workstation, comprising the following steps:
a) positioning the treatment table (1) and the therapy unit (3) relative to one another by
- moving a first arm (14), arranged on the treatment table (1) or the therapy unit (3), from its rest position to its predetermined operational position,
- predetermining the intended position of the therapy unit (3) or the treatment table (1) with the aid of a structural element arranged on the first arm (14) and
- moving the therapy unit (3) or the treatment table (1) into the predetermined intended position,
b) positioning the diagnosis unit (2) relative to the treatment table (1) or the therapy unit (3) by
- moving a second arm (16), arranged on the treatment table (1) or the therapy unit (3), from its rest position to its operational position,
- visually displaying the intended position of the diagnosis unit (2) with the aid of a second structural element arranged on the second arm (16) and
- positioning the diagnosis unit (2) in relation to the second structural element on the basis of a visible marking provided on said diagnosis unit.

11. Method according to Claim 10, wherein, in chronological order, the treatment table (1) and the therapy unit (3) are positioned in relation to one another before the diagnosis unit (2) is positioned relative to the treatment table (1) or the therapy unit (3).

12. Method according to Claim 10 or 11, wherein the treatment table (1) and the therapy unit (3) are fixed in the medical workstation after said treatment table and therapy unit have been positioned.

## Revendications

1. Poste de travail médical, comprenant une unité ( 2 ) de diagnostic, une unité ( 3 ) de thérapie, une table ( 1 ) de traitement et un dispositif de mise en position à l'aide duquel la table ( 1 ) de traitement, l'unité ( 2 ) de diagnostic et l'unité ( 3 ) de thérapie peuvent être mises en position les unes par rapport aux autres, comprenant les caractéristiques suivantes :
a) le dispositif de mise en position comprend un premier et un deuxième bras ( 14, 16 ) qui sont mobiles entre une position de repos et une position de travail pouvant être prescrites, **caractérisé en ce que**
b) le premier bras ( 14 ) sert dans sa position de travail à la mise en position relative entre la table ( 10 ) de traitement et l'unité ( 3 ) de thérapie, est disposé sur la table ( 1 ) de traitement ou sur l'unité ( 3 ) de thérapie et a un premier élément de structure, pour la prescription d'une position de consigne de l'unité ( 3 ) de thérapie ou de la table ( 1 ) de traitement,
c) le deuxième bras ( 16 ) sert dans sa position de travail à la mise en position relative entre l'unité ( 2 ) de diagnostic, d'une part, et la table ( 1 ) de traitement ou l'unité ( 3 ) de thérapie, d'autre part, est disposé sur la table ( 1 ) de traitement ou sur l'unité ( 3 ) de thérapie et a un deuxième élément de structure pour l'indication visuelle d'une position de consigne de l'unité ( 2 ) de diagnostic,
d) l'unité ( 2 ) de diagnostic comprend un repérage visible pour sa mise en position visuelle par rapport au deuxième élément de structure.

2. Poste de travail médical suivant la revendication 1, dans lequel
- le premier bras ( 14 ) est disposé sur la table ( 1 ) de traitement
- l'élément de structure du premier bras ( 14 ) est formé par une surface ( 22 ) de butée, qui sert à la mise en position de l'unité ( 3 ) de thérapie par rapport à la table ( 1 ) de traitement par un contact mécanique.

3. Poste de travail médical suivant la revendication 2, dans lequel l'unité ( 3 ) de thérapie et la surface ( 22 ) de butée du premier bras ( 14 ) sont prévues pour venir directement en butée l'une sur l'autre mécaniquement.

4. Poste de travail médical suivant l'une des revendications précédentes, dans lequel le premier et le deuxième bras ( 14, 16 ) sont disposés sur la table ( 1 ) de traitement.

5. Poste de travail médical suivant la revendication 2, dans lequel l'unité ( 3 ) de thérapie et le premier bras ( 14 ) sont prévus pour venir en butée l'un contre l'autre directement - par butée de la surface ( 22 ) de butée du premier bras ( 14 ) sur une autre surface ( 24 ) de butée du deuxième bras disposé sur l'unité ( 3 ) de thérapie.

6. Poste de travail médical suivant la revendication 1, dans lequel
- le premier bras ( 14 ) est disposé sur l'unité ( 3 ) de thérapie,
- l'élément de structure du premier bras ( 14 ) est conformé pour la prescription visuelle d'une position de consigne de la table ( 1 ) de traitement et
- la table ( 1 ) de traitement comprend un autre repère ( 26 ) visible pour la mise en position visuelle de la table ( 1 ) de traitement par rapport à l'unité ( 3 ) de thérapie.

7. Poste de travail médical suivant la revendication 1 ou 6, dans lequel le premier et le deuxième bras ( 14, 16 ) sont disposés sur l'unité ( 3 ) de thérapie.

8. Poste de travail médical suivant l'une des revendications précédentes, dans lequel l'unité ( 3 ) de thérapie est un lithotripteur.

9. Poste de travail médical suivant l'une des revendications précédentes, dans lequel l'appareil ( 2 ) de diagnostic est un appareil à rayons X à arceau en C.

10. Procédé de mise en position d'une unité ( 3 ) de thérapie, d'une unité ( 2 ) de diagnostic et d'une table ( 1 ) de traitement dans un poste de travail médical, les unes par rapport aux autres, comprenant les stades suivantes :
a) mise en position de la table ( 1 ) de traitement et de l'unité ( 3 ) de thérapie, l'une par rapport à l'autre, en
- faisant passer un premier bras ( 14 ) disposé sur la table ( 1 ) de traitement ou sur l'unité ( 3 ) de thérapie, de sa position de repos dans sa position de travail prescrite,
- à l'aide d'un élément de structure disposé sur le premier bras ( 14 ) prescrivant la position de consigne de l'unité ( 3 ) de thérapie ou de la table ( 1 ) de traitement et
- mettant l'unité ( 3 ) de thérapie ou la table ( 1 ) de traitement en la position de consigne prescrite,
b) mise en position de l'unité ( 2 ) de diagnostic par rapport à la table ( 1 ) de traitement ou à l'unité ( 3 ) de thérapie, en
- faisant passer un deuxième bras ( 16 ) disposé sur la table ( 1 ) de traitement ou sur l'unité ( 3 ) de thérapie, de sa position de repos à sa position de travail,
- à l'aide d'un deuxième élément de structure disposé sur le deuxième bras (16) indiquant visuellement la position de consigne de l'unité ( 2 ) de diagnostic et
- mettant l'unité ( 2 ) de diagnostic en position par rapport au deuxième élément de structure au moyen d'un repère visible présent sur l'unité ( 2 ) de diagnostic.

11. Procédé suivant la revendication 10, dans lequel la mise en position entre la table ( 1 ) de traitement et l'unité ( 3 ) de thérapie s'effectue, considéré dans le temps, avant la mise en position entre l'unité ( 2 ) de diagnostic par rapport à la table ( 1 ) de traitement ou à l'unité ( 3 ) de thérapie.

12. Procédé suivant la revendication 10 ou 11, dans lequel après avoir effectué la mise en position de la table ( 1 ) de traitement et de l'unité ( 3 ) de thérapie, on les immobilise dans le poste de travail médical.
